# EUROPÄISCHE PATENTSCHRIFT

(11) **EP 4 034 185 B1**
(45) Veröffentlichungstag und Bekanntmachung des Hinweises auf die Patenterteilung: **16.04.2025**
(21) Anmeldenummer: 20785457.1
(22) Anmeldetag: 25.09.2020
(51) Int. Cl.: A61M 1/14, A61M 1/16

(54) **VERFAHREN UND SYSTEM ZUR NICHT-ELEKTRISCHEN KOMMUNIKATION BEI WASSERAUFBEREITUNGSANLAGEN ODER MEDIZINGERÄTEN**
METHOD AND SYSTEM FOR NON-ELECTRIC COMMUNICATION IN WATER TREATMENT PLANTS OR MEDICAL APPLIANCES
PROCÉDÉ ET SYSTÈME DE COMMUNICATION NON ÉLECTRIQUE DANS DES INSTALLATIONS DE TRAITEMENT DE L'EAU OU DES APPAREILS MÉDICAUX

(30) Priorität: 27.09.2019 DE 102019126086
(43) Veröffentlichungstag der Anmeldung: 03.08.2022
(73) Patentinhaber: Fresenius Medical Care Deutschland GmbH, 61352 Bad Homburg (DE); Fresenius Medical Care AG, 61352 Bad Homburg (DE); Vivonic GmbH, 63877 Sailauf (DE)
(72) Erfinder: FAROON, Yahya, 48268 Greven (DE); BESSLER, Patrick, 97837 Erlenbach (DE); LINDEMANN, Robert, 65193 Wiesbaden (DE); HELLHUND, Jonas, 60438 Frankfurt (DE); PETERS, Arne, 61352 Bad Homburg (DE); FISCHER, Gerome, 61440 Oberursel (DE)
(74) Vertreter: Herrmann, Uwe
(86) Internationale Anmeldenummer: PCT/EP2020/076829
(87) Internationale Veröffentlichungsnummer: WO 2021/058707

(56) Entgegenhaltungen:
- WO-A1-99/54651
- DE-A1- 19 933 411
- DE-A1- 3 209 189
- DE-A1- 4 037 600
- DE-C1- 10 047 849

## Beschreibung

Die vorliegende Erfindung betrifft eine Kommunikationsvorrichtung, ein Verfahren und ein System zur nicht-elektrischen Kommunikation bei Wasseraufbereitungsanlagen und / oder Medizingeräten.

Im modernen Klinikalltag spielt die Vernetzung einer Mehrzahl von Medizingeräten bzw. Behandlungsmaschinen eine zunehmende Rolle. So wird beispielsweise in größeren Dialysezentren meist eine Vielzahl an Behandlungsmaschinen eingesetzt, die meist an gemeinsame gebäudeseitige Zufuhrleitungen bzw. Wasseraufbereitungsanlagen angeschlossen sind. Die Effizienz des Klinikalltags könnte insbesondere bei derartigen Anwendungen von einer verbesserten Vernetzung der einzelnen Behandlungsgeräte profitieren, da sich auf diese Weise der Betrieb und / oder die Reinigung, die Desinfektion oder die Wartung der einzelnen Behandlungsgeräte miteinander und mit dem Betriebszustand weiterer verbundener Vorrichtungen abstimmen ließe.

Insbesondere bei Blutbehandlungsmaschinen müssen jedoch besondere Vorkehrungen getroffen werden, um sicherzustellen, dass die Patientensicherheit eines an den Flüssigkeitskreislauf einer Blutbehandlungsmaschine angeschlossen Patienten nicht durch elektrische Kommunikationsmodule der Blutbehandlungsmaschinen reduziert wird. Diese Tatsache ist eines der Haupthindernisse bei der Vernetzung von Behandlungsgeräten und insbesondere von Blutbehandlungsgeräten bzw. Dialysemaschinen.

Aus dem Stand der Technik ist beispielsweise aus der DE 199 33 411 A1 eine Wärmemengenerfassung mit Ultraschallübertragung bekannt. Die DE32 091 89 A1 offenbart eine Einrichtung zur Informationsübertragung. Die DE 40 37 600 A1 offenbart eine Fernabfrageeinrichtung für ein Netzwerksystem.

Der vorliegenden Erfindung liegt somit die Aufgabe zugrunde, die aus dem Stand der Technik bekannten Probleme abzumildern oder gar ganz zu beheben. Konkret ist es eine Aufgabe der vorliegenden Erfindung, eine sichere und effiziente Kommunikation zwischen fluidisch miteinander verbundenen Vorrichtungen, wie beispielsweise Behandlungsgeräten, zu ermöglichen.

Diese Aufgabe wird durch einen Kommunikationsvorrichtung nach Anspruch 1, ein Verfahren nach Anspruch 6 und ein System nach Anspruch 11 gelöst. Ein weiterer Aspekt der Erfindung betrifft ein Kit zur Diagnostik eines fluidischen Systems nach Anspruch 5.

Der Kerngedanke der vorliegenden Erfindung liegt hierbei darin, dass die Kommunikation zwischen verschiedenen fluidisch miteinander verbundenen Vorrichtungen nicht-elektrisch erfolgt. Der Grundgedanke der erfinderischen Lösung besteht somit darin, dass Vorrichtungen / Geräte, welche miteinander über eine Flüssigkeitsleitung verbunden sind, die herkömmlicherweise nur zum Transport von Flüssigkeit verwendet wird, über die Leitung bzw. die darin geführte Flüssigkeit nicht-elektrisch, mittels Druckpulsen, miteinander kommunizieren. Die Erfindung umfasst sämtliche fluidisch verbundenen Vorrichtungen, also auch den Fall, dass in den die Vorrichtungen / Geräte verbindenden Leitungen Gas geführt wird.

Dies bietet den Vorteil, dass keine zusätzlichen Kommunikationsleitungen oder Module vorgesehen werden müssen. Weiterhin besteht für den Patienten keine Gefährdung durch elektrischen Strom aufgrund der Kommunikation. Zudem ist die nicht-elektrische Kommunikation im Vergleich zu beispielsweise einer Kommunikation der Geräte über ein Netzwerk weniger anfällig für Angriffe von außen.

Ein erster Aspekt der vorliegenden Erfindung betrifft eine Kommunikationsvorrichtung zur nicht-elektrischen Kommunikation zwischen miteinander fluidisch verbundenen Vorrichtungen, wobei die Kommunikationsvorrichtung dazu ausgelegt ist, in einer Position in einem durch die miteinander fluidisch verbundenen Vorrichtungen gebildeten fluidischen Gesamtsystem angeordnet zu werden und nicht-elektrische Signale in Form von Drucksignalen, zu empfangen und oder / auszusenden, wobei die nicht-elektrische Signale über eine die Vorrichtungen miteinander fluidisch verbindende Leitung übertragen werden, und für einen online Betrieb ausgelegt ist.

Es hat sich als vorteilhaft erwiesen, wenn die Kommunikationsvorrichtung zum Alternativ oder zusätzlich kann die Kommunikationsvorrichtung zum Erfassen von Daten bezüglich mindestens eines Parameters des fluidischen Gesamtsystems und / oder eines Parameters einer in dem fluidischen Gesamtsystem, insbesondere in der die Vorrichtungen miteinander fluidisch verbindenden Leitung, geführten Flüssigkeit ausgelegt sein.

In diesem Fall umfasst die Kommunikationsvorrichtung entsprechende Sensorik zum Erfassen von Daten bezüglich des mindestens einen Parameters, wobei der Parameter vorzugsweise die Leitfähigkeit, Temperatur, das Durchflussvolumen, die optische Durchlässigkeit, Transmissivität, das Absorptionsverhalten, die Trübung, die Turbität oder die Transparenz der Flüssigkeit widerspiegelt und der Parameter des fluidischen Gesamtsystems beispielsweise einen Betriebszustand oder einen Wartungszustand wiederspiegelt. Weiterhin kann der Parameter auch die Dichte der Flüssigkeit oder die Konzentration einzelner Ionen in der Flüssigkeit widerspiegeln. Alternativ oder zusätzlich kann die Kommunikationsvorrichtung auch eine Empfangseinheit aufweisen, welche Daten von einer anderen Instanz / Vorrichtung, insbesondere Daten bezüglich des mindestens einen Parameters, empfangen bzw. einlesen kann. Ebenso wäre es denkbar, dass die Kommunikationsvorrichtung nur eine derartige Empfangseinheit aufweist, aber nicht selbst zum Erfassen von Daten bezüglich des mindestens einen Parameters ausgelegt ist.

Die Kommunikationsvorrichtung ist portabel ausgestaltet.

Die Kommunikationsvorrichtung ist für einen online ausgelegt.

Unter einem online Betrieb ist hierbei zu verstehen, dass die Kommunikationsvorrichtung in den laufenden Betrieb der fluidisch verbundenen Vorrichtungen bzw. des fluidischen Gesamtsystems eingekoppelt ist und beispielsweise Daten bezüglich eines Parameters eines in den die Vorrichtung fluidisch verbindenden Leitungen geführten Fluids erfasst. Weiterhin ist unter einem online Betrieb zu verstehen, dass die Kommunikationsvorrichtung mit weiteren datenverarbeitenden Vorrichtungen in Verbindung steht, sodass die Kommunikationsvorrichtung beispielsweise stets gesteuert werden kann bzw. Daten austauschen kann. In einer nicht beanspruchten Ausführungsform, ist die Kommunikationsvorrichtung beim offline Betrieb dem laufenden Betrieb der fluidisch verbundenen Vorrichtungen bzw. des fluidischen Gesamtsystems getrennt und übermittelt beispielsweise Analyseergebnisse, welche auf der Grundlage der Daten bezüglich des Parameters des Fluids erstellt wurden. Bedarfsweise kann die Kommunikationsvorrichtung in den laufenden Betrieb der fluidisch verbundenen Vorrichtungen bzw. des fluidischen Gesamtsystems eingekoppelt werden. Weiterhin ist unter einem offline Betrieb zu verstehen, dass die Kommunikationsvorrichtung autark arbeitet bzw. nicht mit weiteren datenverarbeitenden Vorrichtungen in Verbindung steht, sodass auf die Kommunikationsvorrichtung in diesem Betriebsmodus nicht von weiteren datenverarbeitenden Vorrichtungen zugegriffen werden kann.

Die Kommunikationsvorrichtung kann dazu ausgelegt sein, kontinuierlich oder intermittierend Daten zu erfassen, zu empfangen und / oder zu senden.

Erfindungsgemäße Kommunikationsvorrichtungen können beispielsweise an einer oder mehreren Positionen in einem fluidischen System mit mehreren fluidisch miteinander verbundenen Vorrichtungen angeordnet werden und so beispielsweise den Betrieb des Systems überwachen und die erfassten Daten an eine zentrale Verarbeitungsstelle weiterleiten oder auch dezentralisiert in den jeweiligen Kommunikationsvorrichtungen verarbeiten.

Erfindungsgemäße Kommunikationsvorrichtungen können aber auch im Rahmen eines Kits zur Diagnostik eines fluidischen Systems zum Einsatz kommen. Ein derartiges Kit umfasst mindestens eine erfindungsgemäße Kommunikationsvorrichtung, vorzugsweise mehrere Kommunikationsvorrichtungen, die beispielsweise ein Benutzer / Service-Techniker zur Diagnostik eines fluidischen Systems beispielsweise an einer Leitung des fluidischen Systems anordnen kann, woraufhin die mindestens eine Kommunikationsvorrichtung Daten erfasst und diese für eine Diagnostik des Wartungs- / Betriebszustand des Systems bereitstellt.

Beispielsweise kann die mindestens eine erfindungsgemäße Kommunikationsvorrichtung des Diagnosekits von einer Wasseraufbereitungsanlage, insbesondere von einer Umkehrosmoseanlage, oder von mindestens einer Vorrichtung, wie z.B. einer Blutbehandlungsmaschine, ausgegebene Signale erfassen. Durch eine Positionierung der mindestens einen erfindungsgemäßen Kommunikationsvorrichtung des Diagnosekits an unterschiedlichen Positionen innerhalb eines fluidischen Gesamtsystems können Fehlerquellen innerhalb des Systems lokalisiert und gezielt behoben werden.

Beispielsweise können von einer Wasseraufbereitungsanlage, wie z.B. einer Anlage zur Bereitstellung von Dialysewasser, über eine zugehörige Wasserleitung Signale ausgegeben werden, welche einen Parameter des bereitgestellten Dialysewassers widerspiegeln. Dieser Parameter kann beispielsweise die Leitfähigkeit, der Druck im Fluidsystem, eine Volumenflussrate oder eine Turbität sein. Die mindestens eine erfindungsgemäße Kommunikationsvorrichtung des Diagnosekits kann beispielsweise an einer bestimmten Position entlang der Wasserleitung angeordnet werden und dort die von der Wasseraufbereitungsanlage ausgegebenen Signale empfangen. Diese Signale können beispielsweise einen Soll-Wert / Referenzwert angegeben, welcher mit einem von der Kommunikationsvorrichtung lokal ermittelten Messwert des selben Parameters verglichen werden kann. So kann z.B. die die Kommunikationsvorrichtung die Leitfähigkeit des Dialysewassers an einer bestimmten aber beliebigen Position in dem fluidischen Gesamtsystem als Ist-Wert ermitteln und es kann somit zu einem Abgleich zwischen dem Soll-Wert und dem Ist-Wert kommen. Wird die Kommunikationsvorrichtung an verschiedenen Positionen in dem fluidischen Gesamtsystem positioniert, so kann eine etwaige Fehlerquelle (beispielsweise eine Kontamination, welche die Leitfähigkeit verändert) in dem fluidischen Gesamtsystem lokalisiert werden. Alternativ oder zusätzlich können auch mehrere Kommunikationsvorrichtungen vorzugsweise gleichzeitig an verschiedenen Stellen des fluidischen Gesamtsystems eingesetzt werden, um etwaige Fehlerquellen zu lokalisieren.

Ein weiterer Aspekt der vorliegenden Erfindung betrifft ein Verfahren zur nicht-elektrischen Kommunikation zwischen miteinander fluidisch verbundenen Vorrichtungen, wobei die nicht-elektrische Kommunikation mittels Drucksignalen über mindestens eine die Vorrichtungen fluidisch verbindende Leitung erfolgt.

Erfindungsgemäß ist an mindestens einer Position in einem durch die fluidisch miteinander verbundenen Vorrichtungen gebildeten fluidischen Gesamtsystem mindestens eine erfindungsgemäße Kommunikationsvorrichtung angeordnet, welche vorzugsweise zum Erfassen von Daten und / oder Empfangen von nicht-elektrischen Signalen und / oder Senden von nicht-elektrischen Signalen ausgelegt ist.

Vorzugsweise erfolgt die nicht -elektrische Kommunikation über eine in der die Vorrichtungen fluidisch verbindenden Leitung geführte Flüssigkeit bzw. ein in der die Vorrichtungen fluidisch verbindenden Leitung geführtes Gas.

Weiterhin hat es sich als vorteilhaft erwiesen, wenn die mindestens eine erfindungsgemäße Kommunikationsvorrichtung zum Erfassen von Daten ausgelegt ist, wobei die von der mindestens einen Kommunikationsvorrichtung erfassten Daten mindestens einen Parameter des fluidischen Gesamtsystems und / oder einen Parameter einer in dem fluidischen Gesamtsystem, insbesondere in der die Vorrichtungen miteinander fluidisch verbindenden Leitung, geführten Flüssigkeit widerspiegeln.

Vorzugsweise wird das erfindungsgemäße Verfahren auf ein fluidisches Gesamtsystem angewandt, welches mindestens eine Wasseraufbereitungsanlage und / oder mindestens eine Behandlungsmaschine, insbesondere eine Blutbehandlungsmaschine, und / oder mindestens eine Leitung, insbesondere eine Zufuhrleitung, eine Ringleitung, eine Wasserleitung, eine Dialysatleitung oder eine Konzentratleitung, aufweist.

Vorzugsweise kommt im Rahmen eines erfindungsgemäßen Verfahrens eine Mehrzahl von erfindungsgemäßen Kommunikationsvorrichtungen zum Einsatz, welche an verschiedenen Positionen des fluidischen Gesamtsystems angeordnet werden.

Ein anderer Aspekt der vorliegenden Erfindung betrifft ein System zur nicht-elektrischen Kommunikation zwischen miteinander fluidisch verbundenen Vorrichtungen, gekennzeichnet durch mindestens eine erfindungsgemäße Kommunikationsvorrichtung, welche an mindestens einer Position des fluidischen Gesamtsystems angeordnet ist und dazu ausgelegt ist, über mindestens eine die Vorrichtungen fluidisch miteinander verbindende Leitung, vorzugsweise einer in dieser Leitung geführten Flüssigkeit, nicht-elektrisch, mittels Drucksignalen zu kommunizieren.

Vorzugsweise weist das System mindestens eine Wasseraufbereitungsanlage und / oder eine Konzentratversorgungsanlage und / oder mindestens eine Behandlungsmaschine, insbesondere eine Blutbehandlungsmaschine, und / oder mindestens eine Leitung, insbesondere eine Zufuhrleitung, eine Ringleitung, eine Wasserleitung, eine Dialysatleitung oder eine Konzentratleitung, auf, welche miteinander fluidisch verbunden sind und so vorzugsweise ein fluidisches Gesamtsystem bilden. Vorzugsweise ist die Wasseraufbereitungsanlage eine Anlage zur Bereitstellung von aufbereitetem Wasser für die Dialyse. Die Wasserleitung dient vorzugsweise der Leitung von aufbereitetem Wasser für die Dialyse, die Konzentratleitung dient vorzugsweise der Leitung von Konzentrat für die Dialyse.

Vorzugsweise ist das im Rahmen der Erfindung zum Einsatz kommende Fluid aufbereitetes Wasser für die Dialyse bzw. Dialysewasser. Unter aufbereitetem Wasser für die Dialyse bzw. Dialysewasser ist hierbei Wasser zu verstehen, das den Erfordernissen der Norm ISO23500 genügt und somit zu Verwendung bei der Dialyse vorgesehen ist. Weiterhin kann die Erfindung aber auch auf Reinwasser, Reinstwasser oder hochgereinigtes Wasser angewandt werden. Ein Beispiel für Reinwasser ist Trinkwasser. Reinstwasser zeichnet sich durch einen höheren Reinheitsgrad aus als Reinwasser und hochgereinigtes Wasser ist noch reiner als Dialysewasser.

Die vorliegenden Erfindung kann auf die Kommunikation verschiedenster fluidisch miteinander verbundener Vorrichtungen angewandt werden. Hierbei können aktive und / oder passive Kommunikationsvorrichtungen in entsprechenden Vorrichtungen wie z.B. Wasseraufbereitungsanlagen, Medizingeräten (Blutbehandlungsgerät, Dialysemaschine etc.), fest oder lösbar angeordnet sein. Die Kommunikationsvorrichtungen sind portabel ausgestaltet und können beispielsweise zum Zweck der Diagnose / Funktionsüberwachung an einer Vorrichtung oder an dem die Vorrichtung fluidisch verbindenden Gesamtsystem angeordnet sein. Beispielhaft werden nachstehend einige Kommunikationsformen / Kommunikationspartner genannt, auf welche die Erfindung angewandt werden kann:
Es kann eine Kommunikation zwischen einer ersten und einer zweiten Umkehrosmoseanlage mittels eines erfindungsgemäßen Kommunikationsverfahrens und / oder mindestens einer oder auch mehrerer erfindungsgemäßer Kommunikationsvorrichtungen erfolgen.

Ebenso ist eine Kommunikation zwischen einer Umkehrosmoseanlage und mindestens einer Kommunikationsvorrichtung zum Zweck der Diagnose / Funktionsüberwachung z.B. der Umkehrosmoseanlage denkbar. Ähnlich dazu kann auch ein Medizingerät mit mindestens einer Kommunikationsvorrichtung zum Zweck der Diagnose / Funktionsüberwachung kommunizieren.

Weiterhin kann die vorliegende Erfindung auf eine Kommunikation zwischen einer Umkehrosmoseanlage und einem Medizingerät (Blutbehandlungsgerät, Dialysemaschine etc.) oder auf eine Kommunikation zwischen mindestens zwei Medizingeräten angewandt werden.

Ein weiterer Anwendungsfall betrifft die Kommunikation zwischen einem Medizingerät und einer lokalen Wasseraufbereitungsvorrichtung. Die lokale Wasseraufbereitungsvorrichtung kann beispielsweise ein Adsorber oder eine ähnliche Vorrichtung zur Auffrischung von verbrauchtem Dialysat in situ sein. Beispielsweise kann das verbrauchte Dialysat an dem Adsorber vorbeigeführt werden und wiederverwendet werden, sodass ein geschlossener Wiederaufbereitungskreislauf gebildet wird. Das Medizingerät und die lokale Wasseraufbereitungsvorrichtung können hierbei in einem gemeinsamen Gehäuse angeordnet sein und / oder fest miteinander verbaut sein.

Ein anderer Anwendungsfall betrifft die Kommunikation zwischen einem Medizingerät und einer lokalen Wasservorbereitungsvorrichtung. Die lokale Wasservorbereitungsvorrichtung stellt hierbei lokal vor der Behandlung die für die Behandlung notwendige Menge ("batch") an Dialysierflüssigkeit bereit, welche aus Dialysewasser und Konzentrat angemischt wird. Das Medizingerät und die lokale Wasservorbereitungsvorrichtung können hierbei in einem gemeinsamen Gehäuse angeordnet sein und / oder fest miteinander verbaut sein.

Bei den vorstehenden genannten Beispielen kann anstelle der Umkehrosmoseanlage auch eine Flash-Destillationsanlage zur Herstellung von Reinstwasser / Reinwasser vorgesehen sein. Auch das Medizingerät und die Flash-Destillationsanlage können hierbei in einem gemeinsamen Gehäuse angeordnet sein und / oder fest miteinander verbaut sein. Die Umkehrosmoseanlage sowie die Flash-Destillationsanlage sind ein Beispiel einer Wasseraufbereitungsanlage.

Weiterhin kann die vorliegende Erfindung auch auf beliebige Kombinationen der vorstehend genannten Kommunikationspartner angewandt werden.

Der Kerngedanke der vorliegenden Erfindung kann in anderen Worten wie folgt formuliert werden:
In der Wassertechnik von Medizingeräten, wie z.B. von Dialysestationen ist es sinnvoll, den Zustand der Installation / der Technik zu kennen und zu überwachen.

Aus diesem Grund werden häufig vernetzte Wassertechnikinstallationen eingesetzt, die dem Anwender Daten wie Temperatur, Leitfähigkeit, Ausbeute, etc. einer Wasseraufbereitungsanlage, wie z.B. einer Umkehrosmose (RO)-Anlage zur Verfügung stellen.

Der Nachteil dieser herkömmlichen Lösung besteht darin, dass nur Daten erhoben und somit bereitgestellt werden, die direkt oder in der unmittelbaren Umgebung der Wasseraufbereitungsanlage bzw. der Umkehrosmose (RO)-Anlage erfasst wurden.

Gemäß einem Aspekt der vorliegenden Erfindung ist es daher vorgesehen, beispielsweise in der Peripherie einer Fluidleitung, wie z.B. einer Ringleitung, verteilte Kommunikationsvorrichtungen vorzusehen, welche an verschiedenen Punkten in dem Fluidleitungssystem lokal Daten sammeln, auswerten und weiterleiten.

Hierbei können die Kommunikationsvorrichtungen mit Aktoren ausgestattet sein, mit deren Hilfe die Kommunikationsvorrichtungen Signale in die Fluidleitung, z.B. Ringleitung / Konzentratleitung einbringen können. Die Kommunikationsvorrichtungen können somit als aktive Sender fungieren.

Dadurch können beispielsweise der Zustand einer Anlage (Wasseraufbereitungsanlage), in der die Fluidleitung fluidisch eingekoppelt ist, sowie Fehler und Ausfälle erkannt und, wenn mehrere Kommunikationsvorrichtungen vorgesehen sind, auch örtlich zugewiesen / lokalisiert werden. Grundsätzlich kann auch lediglich eine Kommunikationsvorrichtung in der Anlage (Wasseraufbereitungsanlage), in der die Fluidleitung fluidisch eingekoppelt ist, vorgesehen sein.

Bei komplexeren Anlagen, beispielsweise Wasseraufbereitungsanlagen in Krankenhäusern oder Dialysezentren kann es sinnvoll sein, mehrere Kommunikationsvorrichtungen vorzusehen. Beispielsweise kann eine Kommunikationsvorrichtung pro Etage vorgesehen sein, eine lokale Detektion eines Fehler auf einer Etage führt dann zum Abschalten der betroffenen Etage, während der Betrieb auf den anderen Etagen unverändert fortgesetzt werden kann.

Die Kommunikationsvorrichtungen können untereinander vernetzt sein. Dies hat den Vorteil, dass neben Fehlern bspw. auch Betriebsabläufe wie Chemische - oder Heißdesinfektion optimiert und den jeweiligen Umgebungsbedingungen angepasst werden können, da die Kommunikationsvorrichtungen die Bedingungen an unterschiedlichen Positionen im Fluidsystem erfassen und so eine lückenlose und umfassende Überwachung des Systems erlauben.

Gemäß einem Aspekt der Erfindung werden Kommunikationsvorrichtungen, welche vorzugsweise Sensoren zum Erfassen von Daten und / oder Aktoren zum Senden von Signalen an der Fluidleitung bzw. Ring - / Konzentratleitung verteilt. Dadurch können Informationen aus der Peripherie zur Auswertung des Zustands der gesamten Wasseraufbereitungsanlage sowie etwaiger damit verbundener Medizingeräte und zur Optimierung im Betriebsablauf herangezogen werden.

Eine Anwendung der vorliegenden Erfindung besteht somit in der Vorhersage erforderlicher Wartungsarbeiten ("predictive maintenance"), wodurch eine reibungslose Funktion einer Wasseraufbereitungsanlage sichergestellt werden kann.

**In** anderen Worten ausgedrückt, kann die vorliegende Erfindung auch wie nachstehend beschrieben werden:
Herkömmlicherweise existiert keine Möglichkeit, die in an eine zentrale Wasserversorgung angeschlossenen Medizingeräten bzw. Behandlungsmaschinen vorhandenen Sensoren (Temperatur, Druck, Leitfähigkeit, etc.) an dem Aufstellort der Behandlungsmaschinen (in situ) auf ihre korrekte Funktionsfähigkeit hin zu überprüfen.

Eine Aufgabe der vorliegenden Erfindung besteht somit darin, eine einfache Möglichkeit zur in-situ Überprüfung der Sensoren von an eine zentrale Wasserversorgung angeschlossenen Medizingeräten bzw. Behandlungsmaschinen zu schaffen.

Eine erfindungsgemäße Kommunikationsvorrichtung weist in einer einfachen Ausführungsform einen Empfänger auf, der von der zentralen Wasserversorgung (oder einem damit verbundenen Transmitter) über die Wasserleitung übertragene Signale empfängt und an eine Schaltung weiterleitet, die die Signale einem Bediener anzeigen und/oder einer Behandlungsmaschine zugänglich machen kann.

Die Signale können Eigenschaften des von der zentralen Wasserversorgung bereitgestellten Wassers beschreiben, bspw. eine am Ausgang der Wasserversorgung gemessene Leitfähigkeit (die ein Maß für die Reinheit des Wassers ist), einen Druck im System, eine Temperatur, einen (Volumen- )Fluss, etc.

Zur Anzeige der empfangenen Signale, die vorzugsweise als Referenz dienen, kann ein Display vorgesehen sein und / oder eine Schnittstelle, die die Signale an ein Gerät mit einem Display weiterleitet (wired/wireless).

Der Behandlungsmaschine können zudem die Signale über eine drahtlose oder bedrahtete Schittstelle zugänglich gemacht werden, wo sie entweder auf einer Anzeige der Behandlungsmaschine angezeigt werden, oder intern mit entsprechenden Werten der in der Maschine vorhandenen Sensoren verglichen werden, z.B. im Rahmen eines ablaufenden Prüfprogramms, wodurch die Funktion der Sensor geprüft wird.

Die Kommunikation zwischen der zentralen Wasserversorgung (bzw. dem damit verbundenen Transmitter) und der Kommunikationsvorrichtung erfolgt bspw. über Druckpulse, die durch die Flüssigkeit in den Leitungen weitergeleitet werden können.

Bei einer Anordnung, in der mehrere Behandlungsmaschinen an eine gemeinsame Ringleitung angeschlossen sind, kann es zur Messung der Parameter der Behandlungsmaschinen bzw. des Leitungssystems erforderlich sein, dass stets nur eine Maschine zu jeder Zeit in Betrieb ist. Dies kann bspw. durch eine entsprechende Kommunikation und Steuerung der Maschinen untereinander sichergestellt sein oder, wenn an jeder der an derselben Ringleitung angeschlossenen Maschinen eine Kommunikationsvorrichtung vorgesehen bzw. angeschlossen ist, die über eine Schnittstelle das jeweilige Behandlungsgerät aktivieren kann, durch eine von der zentralen Wasserversorgung ausgehende Auswahl und Aktivierung des jeweils zu prüfenden Behandlungsgeräts.

Eine weitere Möglichkeit besteht darin, in der Kommunikationsvorrichtung ein steuerbares Ventil vorzusehen, mittels welchem die Wasserversorgung nur für das jeweils zur Prüfung ausgewählte Behandlungsgerät geöffnet/freigeschaltet wird, während die anderen Behandlungsgeräte durch die Ventile der diesen zugeordneten Kommunikationsvorrichtungen von der Wasserversorgung getrennt sind. Die Kommunikationsvorrichtung verfügt in dieser Ausgestaltung über eine eindeutige Adresse, über die sie individuell ansprechbar/adressierbar ist.

Wenn die zentrale Wasserversorgung keine Messwerte zu Eigenschaften des Wassers bereitstellt, kann eine mit entsprechenden Sensoren ausgestattete Kommunikationsvorrichtung in den Wasserkreislauf geschaltet werden. Dies kann an zentraler Stelle oder an einzelnen Ringleitungen eines größeren Systems mit mehreren Ringleitungen erfolgen.

In anderen Worten ausgedrückt, kann die vorliegende Erfindung auch wie folgt wiedergegeben werden:
Ein Kernelement der vorliegenden Erfindung ist die Tatsache, dass verschiedene Geräte eines fluidisch verbundenen Systems physisch miteinander über zumindest eine Flüssigkeitsleitung (beispielsweise für Dialysewasser, Reinstwasser, Reinwasser, hochgereinigtes Wasserund / oder Dialysekonzentrat und / oder Dialysat) in Verbindung stehen und eine nicht-elektrische Kommunikation zwischen den Geräten über diese Flüssigkeitsleitung erfolgt.

Diese nicht-elektrische Kommunikation kann beispielsweise mit Signalen in Form von Druckpulsen erfolgen.

Beispielsweise können erfindungsgemäße Kommunikationsvorrichtungen im Rahmen einer zentralen Wassertechnik- oder Konzentrat-Anlage eingesetzt werden.

Bei dieser Konfiguration ist die Wasseraufbereitungsanlage eine (zentrale) Wassertechnik-Anlage (z.B. Umkehrosmoseanlage [RO] oder Destillationsanlage oder Flash-Destillationsanlage) zur Bereitstellung von hochreinem Wasser (z.B. für die Dialyse) oder eine Konzentrat-Bereitstellungs-Anlage, die z.B. Dialyse-Konzentrat bereitstellt.

Die Anlage verfügt über Kommunikationsvorrichtungen bzw. Mittel zur Kommunikation über die Wasserleitung / Konzentrat-Leitung (die zumindest ein Senden von Signalen ermöglichen), wie beispielsweise Mittel zum Erzeugen von Druckpulsen in der Wasserleitung.

Weiterhin hat die Anlage mindestens eine Ringleitung, die hochreines Wasser von der Wassertechnik-Anlage zu den Entnahmepunkten (Point-of-use) führt, oder eine Konzentratleitung, die von einer Konzentrat-Bereitstellungs-Anlage zu Entnahmepunkten führt.

Weiterhin ist mindestens eine Kommunikationsvorrichtung vorgesehen, die (vorzugsweise lösbar) an der Ringleitung oder Konzentrat-Leitung angebracht ist und über Mittel zum Empfangen von den Signalen der Anlage verfügt, z.B. ein Druckmessmittel.

Die Kommunikationsvorrichtung verfügt vorzugsweise über Mittel zur Auswertung der empfangenen Signale. Weiterhin verfügt die Kommunikationsvorrichtung über Mittel zur Anzeige der ausgewerteten Signale und / oder über Mittel zur drahtgebundenen oder drahtlosen Übertragung der ausgewerteten Signale an eine weitere Instanz (lokal, remote, Leitstelle, Server, Anzeige-Tablet für die Wartung, Behandlungsgerät).

Bei vorteilhaften Weiterbildungen verfügt die mindestens eine Kommunikationsvorrichtung auch über Mittel zum Senden eines Signals.

Gemäß einem anderen Aspekt der Erfindung können auch portable, isoliert agierende Kommunikationsvorrichtungen eingesetzt werden.

Hierbei sind drei alternative vorteilhafte Beispiele denkbar:
Im ersten Beispiel sind zwei Kommunikationsvorrichtungen vorgesehen, die anstelle von Behandlungsgeräten an zwei verschiedenen Stellen an eine fest installierte Flüssigkeitsleitung angeschlossen sind und über Sensorik zur Erfassung von Daten bezüglich der Leitfähigkeit des Wassers, der Volumenflüsse etc. verfügen.

Diese beiden Kommunikationsvorrichtungen können Eigenschaften des Fluidsystems bzw. des Wassersystems (Wasseraufbereitungsanlage und zugehörige Leitungen) messen und miteinander nichtelektrisch über die (Wasser-)Leitung kommunizieren.

Dadurch ist beispielsweise eine Triangulation eines Fehlers, der zu charakteristischen Schallemissionen führt (flatterndes, defektes Ventil; durch eine Leitungsverengung bedingtes Strömungsgeräuch / Pfeifen), möglich. Die Überwachung des Leitungssystems kann also verbessert werden.

Gemäß der ersten Alternative ist eine zentrale Anlage, die Reinwasser und / oder Konzentrat bereitstellt, mit Mitteln zum Senden von Signalen nicht-elektrischer Kommunikation ausgestattet, aber die lokalen Leitungen sind im üblichen Alltagsbetrieb nicht notwendigerweise mit Vorrichtungen zum Empfangen ausgestattet.

Im Diagnosefall / Service-Fall / bei der Inbetriebnahme kann nun eine mobile Kommunikationsvorrichtung an einer Flüssigkeitsleitung angebracht werden, um nicht-elektrische Signale zu empfangen und damit zur Diagnose / zum Service / zur Inbetriebnahme unterstützend beitragen oder zusätzlich auch Signale z.B. bezüglich der gemessenen Daten senden.

Gemäß einer zweiten Alternative dienen die zwei (oder mehr) Kommunikationsvorrichtungen dazu, eine nicht-elektrische Kommunikation zwischen Medizingeräten / Behandlungsgeräten zu ermöglichen. Dazu muss zumindest eine Kommunikationsvorrichtung einen Sender aufweisen, und die andere(n) zumindest jeweils einen Empfänger.

Sofern ein simultaner Betrieb mehrerer Behandlungsgeräte (z.B. in bestimmten Betriebsarten) unerwünscht oder unzulässig ist, kann auf diese Weise eine Abstimmung der Behandlungsgeräte untereinander erfolgen. Die Behandlungsgeräte sind dazu mit den Kommunikationsvorrichtungen bspw. über eine sog. "Legacy-Schnittstelle" oder dgl. verbunden.

Weiterhin kann die vorliegende Erfindung auf Behandlungsgeräte und die zugehörige Infrastruktur ausgedehnt werden.

In dieser Konfiguration ist ein Behandlungsgerät Teil des erfindungsgemäß zur nicht-elektrischen Kommunikation befähigten Systems oder umgekehrt. Dementsprechend verfügt auch ein Behandlungsgerät (z.B. Hämodialysegerät) über Mittel zum Empfangen und/oder zum Senden von nicht elektrischen Kommunikationssignalen über eine Flüssigkeitsleitung.

Die Vorteile der vorliegenden Erfindung bestehen zusammenfassend ausgedrückt darin, dass eine elektrische Verbindung der systembildenden Geräte vermieden werden kann und dass eine ohnehin vorhandene Leitung zusätzlich zum Austausch von Flüssigkeitsmedien auch zum Austausch von nicht-elektrischen Signalen genutzt wird. In anderen Worten wird also eine Kommunikation zwischen Geräten ermöglicht, obwohl diese nicht elektrisch miteinander verbunden sind. Somit wird auch die Gefahr eliminiert, dass Stromschläge oder andere elektrische Störungen im System um sich greifen können. Eine solche Gefahr würde mit einer elektrischen Kopplung einhergehen.

In wieder anderen Worten ausgedrückt umfasst die vorliegende Erfindung somit den Grundgedanken, eine oder mehrere bestehende Fluidleitung(en) und insbesondere flüssigkeitsführende Leitungen zur vorzugsweise aktiven Kommunikation von mittels dieser Fluidleitung(en) verbundenen Geräten zu verwenden.

Nachstehend werden verschiedene erfindungsgemäße Hardware-Konstruktionen erläutert.

Eine einfache Ausführungsform der Erfindung umfasst mindestens eine passive Kommunikationsvorrichtung, die lediglich offline agiert und Daten erhebt, welche sie dann über eine Kommunikationsleitung (auch drahtlos) z.B. an die Wasseraufbereitungsanlage weitergibt.

Eine derartige Kommunikationsvorrichtung ist mit einem Empfänger ausgestattet.

Die Kommunikationsvorrichtung überwacht die Funktion des Gesamtsystems und erfasst somit Daten bezgl. von Wartungszuständen, wie z. B. Lagerschäden an Teilen, Flackern von Ventilen, sowie die Leistung/Drehzahl Pumpe zu Verbrauchsoptimierung/Steuerung.

Eine Auswertung der von der Kommunikationsvorrichtung übermittelten Signale erfolgt in der Wasseraufbereitungsanlage / RO- Anlage und somit zentralisiert bzw. zentral im Zielsystem. In dieser Variante fungiert die Kommunikationsvorrichtung als passiver Empfänger mit einer Datenleitung zur Weitergabe von Signalen. Grundsätzlich könnte auch eine weitere Kommunikationsvorrichtung vorgesehen sein, die zum Senden von Signalen durch die Fluidleitungen des Systems ausgelegt ist.

Betriebsmodi und Wartungszustände können somit erkannt werden und z.B. über eine entsprechende Ausgabe der Kommunikationsvorrichtung oder der Wasseraufbereitungsanlage (z.B. optische Signale wie farbige Leuchten oder auch akustische Signale) angezeigt werden.

Alternativ oder zusätzlich kann die mindestens eine Kommunikationsvorrichtung auch mit einer integrierten Einheit zur Daten-Verarbeitung ausgestattet sein. Die mittels des Empfängers erfassten Daten werden in dieser Variante direkt in der Kommunikationsvorrichtung verarbeitet und die Kommunikationsvorrichtung ist für diesen Zweck mit der entsprechenden Hardware, µC und Ram und Software ausgestattet.

Die Kommunikationsvorrichtung verfügt in dieser Variante über eine Kommunikationsleitung (auch drahtlos) zu irgendeiner beliebigen weiteren Instanz und Betriebsmodi / Wartungszustände des Fluidiksystems können somit erkannt werden und z.B. über eine entsprechende Ausgabe der Kommunikationsvorrichtung (z.B. optische Signale wie farbige Leuchten oder auch akustische Signale) und / oder der weiteren Instanz und / oder der Wasseraufbereitungsanlage angezeigt werden.

Weiterhin bietet diese Variante den Vorteil, dass, auch wenn keine Daten-Leitungen zur elektrischen Datenkommunikation beispielsweise innerhalb eines Dialysezentrums installiert sind, zwischen mehreren Medizingeräten einer Mehrzahl an Medizingeräten Befehle ausgetauscht werden können, wobei die die erfindungsgemäße Kommunikationsvorrichtung als Übersetzer der Befehle bzw. eines bestimmten Befehls in ein Daten- / Status- oder Steuersignal dient. Die Kommunikationsvorrichtung ist auch in diesem nicht beanspruchten Beispiel offline. Es müssen somit keine designierten Kommunikationsleitungen vorgesehen werden, sondern die bereits bestehenden Fluidleitungen können auch für die Kommunikation genutzt werden.

Bestehende Medizingeräte und / oder Wasseraufbereitungsanlagen und / oder Fluidiksysteme können mit mindestens einer erfindungsgemäßen Kommunikationsvorrichtung nachgerüstet werden, welche den Vorteil bietet, dass aktiv Informationen über eine definierte Schnittstelle (Protokoll, I/O digital, analog) ausgetauscht werden können. Unter anderem können die folgenden Kommunikationsschnittstellen / Protokolle verwendet werden: RS232, RS422/485, CAN Bus, CANopen, PROFIBUS, PROFI-NET-RT, Ethernet/IP, Modbus, OPC, Bluetooth, WiFi, SCSI, IDE, ATA, SATA, Firewire, USB, PCI, PCI-E, AGP, GPIP,Rocket link, Thunderbolt, VGA, DVI, HDMI, GSM, UMTS/3G, LTE/4G, 5G, Ethercat, Fernwirkprotokoll nach IEC 60870-5-10x sowie - insbesondere für Nachrüstung - Techniker- und Produktionszugänge wie SPI, I2C, LIN.

Eine weitere erfindungsgemäße Ausführungsvariante sieht mindestens eine passive Kommunikationsvorrichtung vor, die online ist und auf die von außen zurückgegriffen bzw. die von außen gesteuert werden kann.

Gemäß dieser Ausgestaltung umfasst die mindestens eine Kommunikationsvorrichtung einen Empfänger.

Weiterhin weist die mindestens eine Kommunikationsvorrichtung eine Kommunikationsleitung (auch drahtlos) zu einem Gateway auf, welcher Daten in eine Cloud zur Verarbeitung und Auswertung sendet und Feedback an die Wasseraufbereitungsanlage bzw. RO-Anlage, mit der Wasseraufbereitungslage verbundene Medizingeräte / Maschinen oder an die mindestens eine Kommunikationsvorrichtung zurückgibt.

Bestehende Medizingeräte und / oder Wasseraufbereitungsanlagen und / oder Fluidiksysteme können mit mindestens einer erfindungsgemäßen Kommunikationsvorrichtung nachgerüstet werden, welche den Vorteil bietet, dass aktiv Informationen über eine definierte Schnittstelle (Protokoll, I/O digital, analog) ausgetauscht werden können.

Weiterhin bietet auch diese Variante den Vorteil, dass, auch wenn keine Leitungen beispielsweise innerhalb eines Dialysezentrums vorhanden sind, zwischen mehreren Medizingeräten einer Mehrzahl an Medizingeräten Befehle ausgetauscht werden können, wobei die die erfindungsgemäße Kommunikationsvorrichtung als Übersetzer der Befehle bzw. eines bestimmten Befehls in ein Daten- / Status- oder Steuersignal dient. Die mindestens eine Kommunikationsvorrichtung ist in diesem Beispiel online. Die vorstehend beschriebenen passiven Kommunikationsvorrichtungen können im halb-automatischen Inbetriebnahme -Modus mindestens eines Medizingeräts und / oder einer Wasseraufbereitungsanlage eingesetzt werden.

Vorzugsweise kommt hierbei eine Mehrzahl von portablen oder, in eine nicht beansprüchte Ausführungsform, stationären Kommunikationsvorrichtungen zum Einsatz, welche beispielsweise eine Triangulation zur Erfassung einer genauen Position eines Medizingeräts ermöglichen.

Weiterhin erlaubt einer derartige Ausgestaltung auch eine effiziente Fehlerüberwachung und Diagnose, wenn beispielsweise die von verschiedenen Kommunikationsvorrichtungen in einem fluidisch verbundenen Gesamtsystem mit mindestens einem Medizingerät und / oder mindestens einer Wasseraufbereitungsanlage lokal erhobenen Daten miteinander abgeglichen werden.

Bei einem derartigen System mit mehreren Kommunikationsvorrichtungen und / oder mehreren Medizingeräten und / oder mehreren Leitungen / Wasseraufbereitungsanlagen hat es sich als vorteilhaft erwiesen, wenn die verschiedenen Komponenten (Kommunikationsvorrichtungen und / oder Medizingeräte und / oder Leitungen / Wasseraufbereitungsanlagen) jeweils über individuelle Kennungen verfügen und somit individuell adressierbar sind.

Gemäß einem weiteren Aspekt der Erfindung kann mindestens eine aktive Kommunikationsvorrichtung vorgesehen sein, welche entweder online oder, in eine nicht beanspruchte Ausführungsform, offline ist. Eine derartige aktive Kommunikationsvorrichtung umfasst Mittel zum Senden von Signalen nicht-elektrischer Kommunikation, z.B. einen Druckpulsgeber.

Die mittels eines Sensors oder Empfängers erfassten Daten und / oder Steuerungsdaten zur Erzeugung des auszusendenden Signale oder auch sonstige Daten werden in dieser Variante direkt in der Kommunikationsvorrichtung verarbeitet und die Kommunikationsvorrichtung ist für diesen Zweck mit der entsprechenden Hardware µC und Ram und Software ausgestattet.

Die Kommunikationsvorrichtung verfügt in dieser Variante über eine Kommunikationsleitung (auch drahtlos) zu irgendeiner beliebigen weiteren Instanz wie z.B. der Wasseraufbereitungsanlage / RO-Anlage und Betriebsmodi / Wartungszustände des Fluidiksystems können z.B. über eine entsprechende Ausgabe der Kommunikationsvorrichtung (z.B. optische Signale wie farbige Leuchten oder auch akustische Signale) und / oder der weiteren Instanz und / oder der Wasseraufbereitungsanlage angezeigt werden.

Gemäß einem anderen Aspekt der vorliegenden Erfindung sind beispielsweise an einer Leitung des Fluidsystems oder an verschiedenen fluidisch verbundenen Leitungen bzw. an verschiedenen Stellen erfindungsgemäße Kommunikationsvorrichtungen vorgesehen.

Eine jede Kommunikationsvorrichtung umfasst einen Sensor, welcher beispielsweise den Volumenfluss, die Leitfähigkeit, die Transparenz, die Temperatur oder einen sonstigen Parameter eines in der Leitung fließenden Fluids erfasst. Eine jede Kommunikationsvorrichtung ist zudem dazu ausgelegt, die erfassten Daten über dasselbe Fluid an mindestens eine andere Kommunikationsvorrichtung und / oder die Wasseraufbereitungsanlage und / oder eine beliebige weitere Instanz weiterzuleiten. Beispielsweise erfolgt die nicht-elektrische Signalübermittlung mittels eines Druckpulsgeber.

In anderen Worten dient das Messmedium (also ein Fluid wie z.B. Dialyse-Konzentrat oder Reinwasser, das Gegenstand einer Erfassung / Messung von Parametern ist, z.B. Temperatur) erfindungsgemäß gleichzeitig als Kommunikationsmedium. Aufgrund der verteilten Anordnung der Kommunikationsvorrichtungen und somit Sensoren werden zielgerichtet und lokal in kritischen Punkten des Gesamtsystems, z.B. an einem Point of Use, Informationen erhoben. Beispielsweise kann bei der Heißdesinfektion die Temperatur des Fluids und bei der chemischen Desinfektion die Leitfähigkeit des Fluids an mehreren Punkten des fluidischen Gesamtsystems erfasst werden.

Dies ermöglicht eine besonders gute Optimierung der Steuerungsvorgänge sowie der Anlagemodi bezüglich beispielsweise der eingesetzten Temperaturen, Energie, Spülzyklen und oder Spülmengen etc. Wie vorstehend beschrieben kann die Daten-verarbeitung entweder dezentralisiert in einer jeden Kommunikationsvorrichtung oder zentralisiert erfolgen. Dadurch kann z.B. vorteilhaft in einem System mit mehreren Medizingeräten, die einer regelmäßigen Desinfektion - z.B. mit Heißwasser - bedürfen so gesteuert und kommuniziert werden, dass nicht alle Medizingeräte gleichzeitig desinfiziert werden, sodass die Anforderung an die Leistung des Desinfektionsvorgangs oder der Desinfektionsmittel reduziert werden kann. So kann beispielsweise sequentiell jeweils ein Teil der Maschinen desinfiziert werden, weil dann nur eine geringere Menge Desinfektionsmittel zu einem bestimmten Zeitpunkt bereitgestellt werden muss, z.B. Heißwasser. Im Fall von Heißwasser könnte dann ein Tank verkleinert oder die Heizleistung einer Heizvorrichtung reduziert werden.

Die einzelnen Kommunikationsvorrichtungen können die erfassten Daten zusätzlich auch über eine Kommunikationsleitung (auch drahtlos) weitergeben und Betriebsmodi / Wartungszustände des Fluidiksystems können z.B. über eine entsprechende Ausgabe der Kommunikationsvorrichtung (z.B. optische Signale wie farbige Leuchten oder auch akustische Signale) und / oder der weiteren Instanz und / oder der Wasseraufbereitungsanlage angezeigt werden.

Die von den Kommunikationsvorrichtungen erfassten Daten können miteinander und oder mit anderweitig abgelegten Referenzwerten verglichen werden, um die Betriebsmodi / Wartungszustände zu bestimmen.

Gemäß einem anderen Aspekt der Erfindung ist es vorgesehen, mindestens zwei erfindungsgemäße Kommunikationsvorrichtungen gemäß einer der vorstehenden Varianten im Rahmen der Funktionsüberwachung eines Medizingeräts, wie beispielsweise einer Blutbehandlungsmaschine, einzusetzen.

Beispielsweise können die mindestens zwei erfindungsgemäßen Kommunikationsvorrichtungen an einer Zu- und einer Ableitung des Medizingeräts oder an zwei sonstigen Stellen in der Fluidik des Medizingeräts eingesetzt werden. Im Falle einer Blutbehandlungsmaschine können die mindestens zwei erfindungsgemäßen Kommunikationsvorrichtungen jeweils in einer Leitung der RO-Anlage und einer KonzentratLeitung angeordnet sein.

Mittels der mindestens zwei erfindungsgemäßen Kommunikationsvorrichtungen kann die Funktion der Wassertechnik des Medizingeräts überwacht und bewertet werden. Weiterhin kann auf einen jeweiligen Betriebsmodus des Medizingeräts zurückgeschlossen werden. Weiterhin kann zur Erkennung von Fehlerfällen eine Plausibilitätsüberprüfung der von den mindestens zwei erfindungsgemäßen Kommunikationsvorrichtungen erfassten Daten erfolgen.

Beispielsweise kann an einer Zu- und einer Ableitung des Medizingeräts, z.B. einer Dialysemaschine, jeweils mit einer Kommunikationsvorrichtung ein Parameter des Fluids gemessen werden und aus einem Vergleich der Messwerte miteinander und / oder mit entsprechenden Referenzwerten auf die Funktion der Medizingeräts beschlossen werden. Beispielsweise kann mittels einer ersten Kommunikationsvorrichtung die Turbität von frischer Dialysierflüssigkeit vor der Einleitung in den Dialysator gemessen werden. Zudem kann mittels einer zweiten Kommunikationsvorrichtung die Turbität von verbrauchter Dialysierflüssigkeit stromabwärts des Dialysators gemessen werden. Eine erhöhte Turbität der verbrauchten Dialysierflüssigkeit relativ zu der frischen Dialysierflüssigkeit kann beispielsweise auf eine Leckage im Dialysator und somit auf Spuren von Blut in der verbrauchten Dialysierflüssigkeit hindeuten.

Alternativ oder zusätzlich kann aber auch eine zentrale Steuerungseinheit, eine Wasseraufbereitungsanlage oder das Medizingerät bzw. die Dialysemaschine Signale betreffend einer Soll-Temperatur des Dialysewassers bzw. der Dialysierflüssigkeit mittels der erfindungsgemäßen nicht-elektrischen Kommunikation ausgeben. An verschiedenen Positionen des fluidischen Gesamtsystems, z.B. an der Dialysemaschine, angeordnete Kommunikationsvorrichtungen können daraufhin lokal die Temperatur des Dialysewassers bzw. der Dialysierflüssigkeit erfassen und die entsprechenden Messwerte an die Dialysemaschine senden und / oder lokal auswerten und / oder an eine zentrale Steuereinheit, eine weitere Analysevorrichtung oder eine Anzeigeeinheit weiterleiten. Unter Dialysierflüssigkeit ist hierbei mit Konzentrat versetztes Dialysewasser zu verstehen, welches die für die Behandlung erforderlichen Bestandteile in angebrachter Menge enthält.

Ist beispielsweise das Medizingerät eine Blutbehandlungsmaschine (Dialysemaschine) und eine erfindungsgemäße Kommunikationsvorrichtung erfasst, dass kein Dialysewasser bzw. Permeat verbraucht wird, während die andere Kommunikationsvorrichtung erfasst, dass Konzentrat verbraucht wird, so deutet das auf einen Fehlerfall und insbesondere auf eine Leckage hin. Wird hingegen durch die Kommunikationsvorrichtung erfasst, dass Dialysewasser bzw. Permeat verbraucht wird und durch die andere Kommunikationsvorrichtung erfasst, dass gleichzeitig Konzentrat verbraucht wird, so deutet dies darauf hin, dass die Dialysemaschine zur Durchführung einer Behandlung und somit zur Bereitstellung von Dialysierflüssigkeit betrieben wird. Die von den Kommunikationsvorrichtungen erfassten Daten können somit miteinander und oder mit anderweitig abgelegten Referenzwerten verglichen werden, um die Fehlerfälle / Betriebsmodi / Wartungszustände zu bestimmen.

Ein weiterer Aspekt der Erfindung betrifft die Nachrüstung von Bestandsgeräten, insbesondere Wasseraufbereitungsanlagen und Medizingeräten, mit erfindungsgemäßen Kommunikationsvorrichtungen, mittels welcher vorteilhafterweise aktiv Informationen über definierte Schnittstellen (Protokoll, I/O digital, analog etc. sowie die weiteren vorstehend genannten Schnittstellen) ausgetauscht werden können.

Beispielsweise kann die vorliegende Erfindung auch als Messequipment für Service-Techniker zur schnellen Diagnose /Sicherheitstechnische Kontrolle (STK) von Leitungen, insbesondere Ringleitungen (Leckage) oder der daran abgeschlossenen Medizingeräte und / oder Wasseraufbereitungsanlagen dienen.

Erfindungsgemäße Kommunikationsvorrichtungen können vorteilhafterweise als lokale Auswerteinheiten ähnlich einer Service Software eingesetzt werden. Mindestens eine erfindungsgemäße Kommunikationsvorrichtung wird in diesem Fall als Diagnosegerät eingesetzt. Es kann auch ein Diagnosekit mit mehreren erfindungsgemäßen Kommunikationsvorrichtungen vorgesehen sein.

Beispielsweise bringt ein Service-Techniker eine (oder mehrere) portable erfindungsgemäße Kommunikationsvorrichtung mit in ein Behandlungszentrum / Dialysezentrum (das nicht mit erfindungsgemäße Kommunikationsvorrichtungen ausgestattet sein muss) und ordnet die erfindungsgemäße Kommunikationsvorrichtung z.B. an einen Point of Use in einem Behandlungsraum an einer Ringleitung einer Wasseraufbereitungsanlage an, wo sonst ein Medizingerät / eine Behandlungsmaschine angeschlossen wäre.

Mit einer derart angeschlossenen erfindungsgemäßen Kommunikationsvorrichtung kann bereits eine Diagnostik der Ringleitung durchgeführt werden. Alternativ oder zusätzlich könnte der Service-Techniker einen an / von der erfindungsgemäßen Kommunikationsvorrichtung gemessenen Wert mit dem Messwert desselben Parameters einer benachbart angeschlossenen Behandlungsmaschine vergleichen, um so Rückschlüsse auf Betriebsmodi, Betriebszustände, Wartungszustände und Fehlerfälle ziehen zu können.

Bei Weiterbildungen der Erfindung gibt es weitere Instanzen, die der Techniker bei der Diagnose einbezieht, wie z.B. eine sendende RO-Anlage und/oder eine weitere erfindungsgemäße Kommunikationsvorrichtung, wie dies vorstehend beschrieben ist.

Bei dieser Ausgestaltung der Erfindung kann eine Parametrierung/Einstellung an den verteilten erfindungsgemäßen Kommunikationsvorrichtungen erfolgen. Die Verwendung mehrerer erfindungsgemäßer erfindungsgemäßen Kommunikationsvorrichtungen zur Systemdiagnose ermöglicht einen schnellen Überblick über den Gesamtaufbau des Behandlungszentrums sowie die Infrastruktur bzw. Art der Gerät und Betriebszustände.

Für zentrale Wartungsmaßnahmen (z.B. Desinfektion der RO-Anlage) kann der Zustand einzelner Medizingeräte mittels der mindestens einen Kommunikationsvorrichtung abgefragt werden. Daraufhin können bestimmte Betriebsmodi gezielt untersagt werden, z.B. zur Unterbrechung einer laufenden Behandlung oder zum Ausspülen können einzelne Geräte angeschaltet oder ausgeschaltet / gesteuert werden und Daten können abgefragt werden.

Da all dies mittels der mindestens einen Kommunikationsvorrichtung bzw. der Mehrzahl an Kommunikationsvorrichtungen ermittelt wird, werden die Laufwege für den Service-Techniker reduziert. Daraufhin kann eine gezielte Schaltung von Geräten in bestimmte Servicemodi oder zur max. Auslastung für die Parametrierung zentraler Anlagen (RO, Konzentrat) erfolgen.

Es hat sich weiterhin als vorteilhaft erwiesen, wenn erfindungsgemäße Kommunikationsvorrichtungen portabel / beweglich / tragbar ausgeführt sind.

Portable Kommunikationsvorrichtungen können zudem vorzugsweise an eine Wasseraufbereitungsanlage, insbesondere an eine RO-Anlage wie ein Medizingerät bzw. ein Behandlungsgerät angeschlossen werden. Erfindungsgemäße Kommunikationsvorrichtungen können zudem passiv oder aktiv sein und für einen online oder, in eine nicht beanspruchte Ausführungsform, einen offline Betrieb ausgelegt sein.

Gemäß einem anderen Aspekt der Erfindung wird die Erfindung auf ein System mit einer RO-Anlage, einem Behandlungsgerät und anderen Infrastrukturgeräten ( wie z.B. Klimaanlagen, Heizsystemen, Wasservorbehandlungsanlagen etc. ) angewandt.

Gemäß einer Ausgestaltung der Erfindung umfasst das Behandlungsgerät Komponenten einer aktiven erfindungsgemäßen Kommunikationsvorrichtung. In anderen Worten ist die Funktion der erfindungsgemäßen Kommunikationsvorrichtung in dem Behandlungsgerät integriert.

Die aktive Kommunikationsvorrichtung ist, wie vorstehend beschrieben entweder online oder, in eine nicht beanspruchte Ausführungsform, offline.

Eine aktive Kommunikationsvorrichtung umfasst Mittel zum Senden von Signalen nicht-elektrischer Kommunikation, z.B. einen Druckpulsgeber.

Die mittels eines Empfängers erfassten Daten und / oder Steuerungsdaten zur Erzeugung des auszusendenden Signale oder auch sonstige Daten werden in dieser Variante vorzugsweise direkt in der Kommunikationsvorrichtung verarbeitet und die Kommunikationsvorrichtung ist für diesen Zweck mit der entsprechenden Hardware µC und Ram und Software ausgestattet.

Die Kommunikationsvorrichtung verfügt in dieser Variante über eine Kommunikationsleitung (auch drahtlos) zu irgendeiner beliebigen weiteren Instanz wie z.B. der Wasseraufbereitungsanlage / RO-Anlage und Betriebsmodi / Wartungszustände des Fluidiksystems können z.B. über eine entsprechende Ausgabe der Kommunikationsvorrichtung (z.B. optische Signale wie farbige Leuchten oder auch akustische Signale) und / oder der weiteren Instanz und / oder der Wasseraufbereitungsanlage angezeigt werden.

Das Behandlungsgerät kann zusätzliche Sensoren zum Erfassen der Leitfähigkeit eines Fluids, der Temperatur, Dichte, des Flussvolumens etc. aufweisen. Auf diese Weise werden zusätzliche Informationen bezüglich beispielsweise eines Desinfektionsvorgangs, zur Hygiene oder auch zur Medienbeschaffenheit geliefert. Beispielsweise kann das Behandlungsgerät bei einer Heißdesinfektionen Messwerte bezüglich der in der Internfluidik des Behandlungsgeräts herrschenden Temperaturen liefern oder bei einer chemischen Desinfektion Messwerte bezüglich der Leitfähigkeit des in der Internfluidik des Behandlungsgeräts fließenden Fluids liefern. Sind mehrere Behandlungsgerät vorgesehen, beispielsweise in einem Dialysezentrum, so kann jedes Behandlungsgerät die entsprechenden Messwerte an einen zentrale Steuerung senden. Auf der Grundlage der übermittelten Werte können dann die Einstellungen / Bedingungen bei jedem Behandlungsgerät individuell optimiert werden, beispielsweise durch eine gezielte lokale Anpassung der Temperatur oder eine gezielte lokale Zudosierung von Desinfekionsmittel.

Diese Ausgestaltung bietet somit den Vorteil, dass auch eine zentrale Messung der Parameter erfolgen kann, sodass eine derartige Steuerung / Regelung ausgeführt werden kann, dass an einem definierten Point of Use stets optimale Bedingungen erreicht werden. Zudem kann eine Koordinierung und / oder Bewertung aller Anforderungen verschiedener Verbraucher erfolgen.

Alternativ oder zusätzlich kann es gemäß einem anderen Aspekt der Erfindung bei einem derartigen System auch vorgesehen sein, dass eine passive erfindungsgemäße Kommunikationsvorrichtung in Kombination mit einer aktiv sendenden Behandlungsmaschine im Rahmen eines Fluidsystems zum Einsatz kommt.

In diesem Fall umfasst das Behandlungsgerät Mittel zum Senden von Signalen nicht-elektrischer Kommunikation, z.B. einen Druckpulsgeber.

Die passive Kommunikationsvorrichtung erfasst, wie vorstehend beschrieben, mittels eines Empfängers die Signale des Behandlungsgeräts.

Diese Ausgestaltung bietet den Vorteil, dass Prozesse und insbesondere die Verbrauchssteuerung (Energie, Wasser, Desinfektionsmittel etc.) optimiert werden können. So kann die Erfindung nach dieser Ausgestaltung vorzugsweise im Rahmen eines fluid on demand -Systems umgesetzt werden.

In einer weiteren Variante sind sowohl die erfindungsgemäße Kommunikationsvorrichtung, als auch das Behandlungsgerät aktiv und senden somit nicht-elektrische Signale aus. Das Behandlungsgerät und / oder die Kommunikationsvorrichtung weisen hierbei jeweils einen Empfänger und / oder Mittel zum Senden von Signalen (z.B. einen Druckpulsgeber) auf.

Beispielsweise könnte die RO-Anlage in Form von Druckpulsen (ein Puls pro Mikrosiemens) einen Leitwert an die fluidisch verbundenen Behandlungsmaschinen kommunizieren. Ein Problem könnte hierbei entstehen, wenn die Behandlungsmaschine eine Wassereingangskammer hat, die einen Transfer von Druckpulsen aus dem Ring zu Kommunikationszwecken unbrauchbar macht.

Es wäre denkbar, mindestens eine erfindungsgemäße Kommunikationsvorrichtung in der Nähe mindestens einer Behandlungsmaschine anzuordnen, welche die Impulse von der RO-Anlage empfängt und an die Behandlungsmaschine weitergibt. Alternativ dazu wäre es denkbar, dass die Behandlungsmaschine ein Mikrofon (z.B. für Körperschall) an der Ringleitung hat, sodass trotz einer etwaigen Wassereingangskammer der Behandlungsmaschine eine effiziente Kommunikation zwischen der RO-Anlage und Behandlungsmaschine erfolgen kann, da die Wassereingangskammer kein Problem für die Schallaufnahme darstellen kann.

Zusammenfassend ist festzuhalten, dass die vorliegende Erfindung die folgenden Vorteile bietet:
Es erfolgt eine nicht-elektrische Kommunikation über ein Fluid bzw. eine Flüssigkeit in einer Leitung, wie z.B. eine Dialysewasserleitung. Dies ist besonders im Medizinbereich sehr vorteilhaft, da elektrische Kommunikation insbesondere im Kontext von flüssigkeitsführenden Leitungen bzw. Wasseraufbereitungsanlagen suboptimal und somit zu vermeiden ist.

Weiterhin ist beispielsweise bei einer Anwendung der Erfindung auf eine Blutbehandlungsmaschine oder Dialysemaschine eine Kommunikation über die Dialysekonzentratleitung (und nicht nur über Leitungen des Wasseraufbereitungssystems (Water Treatment)) möglich. Durch die Integration des Konzentrats und einer Konzentratringleitung entsteht somit ein möglicher zweiter Kommunikationsweg zwischen den Komponenten des Fluidsystems.

Ein weiterer Vorteil der Erfindung besteht in einer sehr geschützten Kommunikation, das die Fluidleitungen und somit erfindungsgemäß die Kommunikationswege medizinischer Fluidleitungssysteme keine Verbindung zur Außenwelt aufweisen, da beispielsweise die Wasserversorgung durch freien Einlauf (EN 1717) von der Außenwelt entkoppelt/getrennt ist.

Weiterhin besteht die Möglichkeit, eine zusätzliche Schnittstelle zur verschlüsselten Übertragung mit anderen Medien (WLAN, eine Sequenz zeitlich aufeinanderfolgender QR-Codes (QR-Stream), Elektrisch) zusätzlich zu der nicht-elektrischen Kommunikation vorzusehen.

Die vorliegende Erfindung ermöglicht somit eine aktive Einkoppelung / Transport von Informationen in dem in einer Leitung geführten Fluid / Flüssigkeit. Diese Information betreffen beispielsweise die Entnahme, Entnahmemenge, den Betriebszustand, die Anforderung von Medium, einen Information Standby, Statusinformationen der angeschlossen Geräte (uni- und bidirektional), die Start / Stopp-Kommunikation, die Erfassung von Konzentratsorten zur Konzentratwahl etc. So können Fragestellungen beantwortet werden, wie z.B. ob sich das am HD-Gerät gewählte Konzentrat in der Ringleitung befindet.

Weiterhin kann in dem erfindungsgemäßen Verfahren bzw. der Vorrichtung eine Sicherheitsfunktion zur Blockierung / Freigabe bestimmter Betriebsmodi ja nach Medium oder Betriebsphase (Desinfektion/ Versorgen etc.) implementiert sein.

Die vorliegende Erfindung ermöglicht eine effiziente Überwachung / Monitoring von Veränderungen der Installation/Geräte.

Zudem ermöglicht es die vorliegende Erfindung zu prüfen, welches Medium in einer Leitung ist (Desinfektionsmittel, Dialyse-Konzentrat, Wasser, Dialysewasser, Heißwasser) und somit einen bestimmten Betriebszustand (z.B. Desinfektion, Versorgen) eines Medizingeräts bzw. einer Wasseraufbereitungsanlage zu erkennen und zu gewährleisten.

Die vorliegende Erfindung ermöglicht zudem eine Temperaturüberwachung des Mediums / Fluids in einer Leitung bzw. einer Ringleitung. Die Temperatur des Mediums beeinflusst beispielsweise dessen Dichte (bekannte Ringleitungskonstellation) und es wird im Rahmen einer Temperaturüberwachung ein entsprechendes Signal von einer erfindungsgemäßen Kommunikationsvorrichtung ausgegeben.

Weiterhin ermöglicht die vorliegende Erfindung ein einfaches Vermessen einer Leitung, z.B. einer Ringleitung, an welcher mehrere Behandlungsmaschinen angeordnet sind, zur Charakterisierung für Installation und Servicezwecke, sowie zur Optimierung des Betriebes. Es können somit beispielsweise optimale Spülmengen, Desinfektionsmittelmengen, Heißwassermengen, Entlüftungszyklen etc. eingestellt werden, während durch die erfindungsgemäßen Kommunikationsvorrichtungen lokal und gezielt die Funktion des fluidischen Gesamtsystems überwacht wird. Auch hinsichtlich der Anschlussanzahl sowie hinsichtlich des Orts / der Position von Entnahmestellen kann das fluidische System optimiert werden. Weiterhin kann die vorliegende Erfindung zur Erkennung von Verunreinigungen (z. B. Luftblasen, bakterielles Wachstum usw.) in Leitungen / den Ringleitungen eingesetzt werden.

Zusätzlich dazu bietet die vorliegende Erfindung eine gute Möglichkeit zur Leckageerkennung / Dichtheitsprüfung während der Installation und in der Betriebsphase des fluidischen Systems.

Weitere Vorteile, Merkmale und Effekte der vorliegenden Erfindung ergeben sich aus der nachstehenden Beschreibung unter Bezugnahme auf die Figuren. In den Figuren bezeichnen dieselben Bezugszeichen gleiche oder ähnliche Bauteile. Hierbei zeigt:
Fig. 1 den allgemeinen Aufbau einer Flüssigkeitsversorgung für mehrere Blutbehandlungsgeräte mittels einer Ringleitung, wie sie häufig in Dialysezentren zum Einsatz kommt.
Fig. 2 den Aufbau aus Fig. 1 mit entlang der Ringleitung angeordneten Kommunikationsvorrichtungen.
Fig. 3 zeigt eine erfindungsgemäße Kommunikationsvorrichtung, welche an einer Leitung zwischen einer Blutbehandlungsvorrichtung und einer Wasseraufbereitungsanlage bzw. RO-Anlage 3 angeordnet ist und Schallwellen 6 aussendet.
Fig. 4 zeigt den grundsätzlichen Aufbau einer erfindungsgemäßen Kommunikationsvorrichtung.

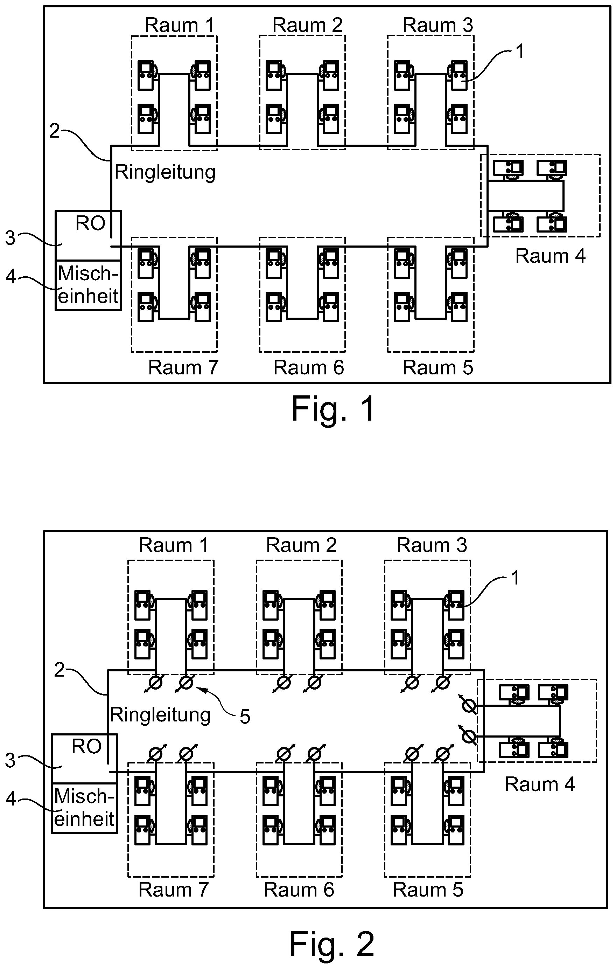

Fig. 1 zeigt mehrere Blutbehandlungsvorrichtungen 1, welche in mehreren verschiedenen Räumen stehen und miteinander über eine Ringleitung 2 miteinander sowie mit einer Wasseraufbereitungsanlage / RO-Anlage 3 und einer Mischeinheit 4 zum Erzeugen von Dialyselösung aus Konzentrat fluidisch verbunden sind und somit ein fluidisches Gesamtsystem bilden.

Fig. 2 zeigt den Aufbau aus Fig. 1 mit entlang der Ringleitung 2 angeordneten Kommunikationsvorrichtungen 5. Die Kommunikationsvorrichtungen 5 ermöglichen in dieser Anordnung beispielsweise das Erfassung von Daten / Messen von Daten bezüglich des Fluids / der Flüssigkeit in der Ringleitung 2, beispielsweise das Durchflussvolumen, die Temperatur oder die Leitfähigkeit. Die Anordnung der Kommunikationsvorrichtungen 5 ermöglicht somit das Erfassen von Daten für einzelne Gruppen von Blutbehandlungsvorrichtungen 1, ohne dass der Betrieb der anderen Blutbehandlungsvorrichtungen 1 hierdurch beeinträchtigt wird oder die anderen Geräte in einen bestimmten Betriebsmodus versetzt werden müssen, der beispielsweise eine (zentrale) Messung beeinträchtigen könnte.

Besonders bei derartigen Anordnungen mit mehreren Vorrichtungen (Behandlungsgeräten, Wasseraufbereitungsanlagen etc.) und mehreren Kommunikationsvorrichtungen ist es sinnvoll, wenn sowohl die Vorrichtungen als auch die Kommunikationsvorrichtungen jeweils über individuelle Kennungen verfügen und somit individuell adressierbar sind.

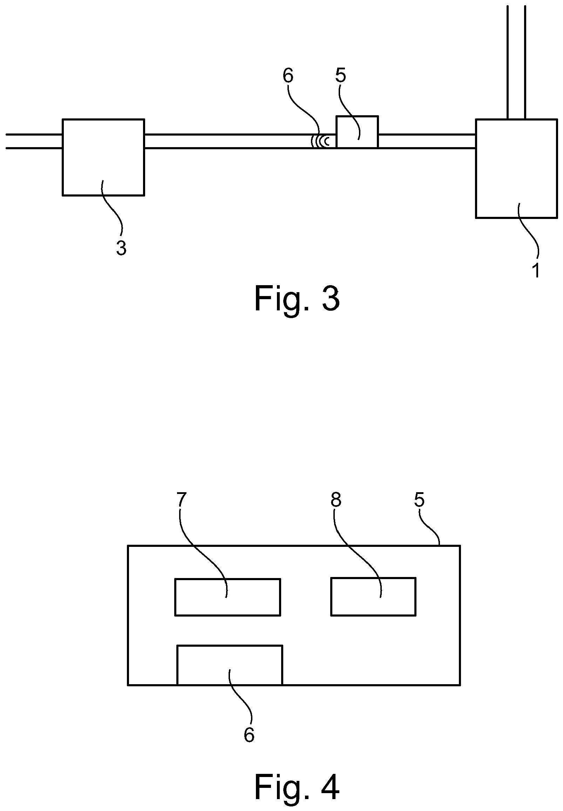

Fig. 3 zeigt eine erfindungsgemäße Kommunikationsvorrichtung 5, welche an oder in einer Leitung, beispielsweise einer Ringleitung 2, zwischen einer Blutbehandlungsvorrichtung 1 und einer Wasseraufbereitungsanlage bzw. RO-Anlage 3 angeordnet ist und akustisch durch das Aussenden akustischer Signale in das in der Leitung fließende Flüssigmedium kommuniziert, wie dies durch die Schallwellen 6 verdeutlicht wird.

Fig. 4 zeigt den grundsätzlichen Aufbau einer erfindungsgemäßen Kommunikationsvorrichtung 5. Die Kommunikationsvorrichtung 5 weist beispielsweise einen Sensor / eine Sensorik 6 zum Erfassen mindestens eines Parameters des fluidischen Gesamtsystems, welches durch die Fluidleitungen und die daran gekoppelten Vorrichtungen gebildet wird, auf.

Weiterhin weist die Kommunikationsvorrichtung 5 einen Empfänger 7 zum Empfangen nicht-elektrischer Signale auf. Zudem weist die Kommunikationsvorrichtung 5 einen Sender 8 zum Senden nicht-elektrischer Signale auf.

## Patentansprüche

1. Kommunikationsvorrichtung (5) zur nicht-elektrischen Kommunikation zwischen miteinander fluidisch verbundenen Vorrichtungen, **dadurch gekennzeichnet, dass** die Kommunikationsvorrichtung dazu ausgelegt ist, in einer Position in einem durch die miteinander fluidisch verbundenen Vorrichtungen gebildeten fluidischen Gesamtsystem angeordnet zu werden und nicht-elektrische Signale in Form von Drucksignalen, zu empfangen und / oder auszusenden, wobei die nicht-elektrische Signale über eine die Vorrichtungen miteinander fluidisch verbindende Leitung übertragen werden, und die Kommunikationsvorrichtung portabel und für einen online Betrieb ausgelegt ist.

2. Kommunikationsvorrichtung nach Anspruch 1, **dadurch gekennzeichnet, dass** die Kommunikationsvorrichtung zum Senden nicht-elektrischer Signale einen Druckpulsgeber aufweist.

3. Kommunikationsvorrichtung nach einem der vorhergehenden Ansprüche, **dadurch gekennzeichnet, dass** die Kommunikationsvorrichtung weiterhin zum Erfassen von Daten bezüglich mindestens eines Parameters des fluidischen Gesamtsystems und / oder eines Parameters einer in dem fluidischen Gesamtsystem, insbesondere in der die Vorrichtungen miteinander fluidisch verbindenden Leitung, geführten Flüssigkeit ausgelegt ist.

4. Kommunikationsvorrichtung nach Anspruch 3, **dadurch gekennzeichnet, dass** die Kommunikationsvorrichtung Sensorik zum Erfassen von Daten bezüglich des mindestens einen Parameters aufweist, wobei der Parameter vorzugsweise die Leitfähigkeit, Temperatur, das Durchflussvolumen, die Turbität oder die Transparenz der Flüssigkeit widerspiegelt und der Parameter des fluidischen Gesamtsystems beispielsweise einen Betriebszustand oder einen Wartungszustand wiederspiegelt.

5. Kit zur Diagnostik eines fluidischen Systems umfassend mindestens eine Kommunikationsvorrichtung, vorzugsweise mehrere Kommunikationsvorrichtungen, nach einem der vorhergehenden Ansprüche.

6. Verfahren zur nicht-elektrischen Kommunikation zwischen miteinander fluidisch verbundenen Vorrichtungen, **dadurch gekennzeichnet, dass** die nicht-elektrische Kommunikation mittels Drucksignalen über mindestens eine die Vorrichtungen fluidisch verbindende Leitung erfolgt, wobei an mindestens einer Position in einem durch die fluidisch miteinander verbundenen Vorrichtungen gebildeten fluidischen Gesamtsystem mindestens eine Kommunikationsvorrichtung nach einem der Ansprüche 1 bis 4 angeordnet ist, welche vorzugsweise zum Erfassen von Daten und / oder Empfangen von nicht-elektrischen Signalen und / oder Senden von nicht-elektrischen Signalen ausgelegt ist.

7. Verfahren nach Anspruch 6, **dadurch gekennzeichnet, dass** die nicht -elektrische Kommunikation über eine in der die Vorrichtungen fluidisch verbindenden Leitung geführte Flüssigkeit erfolgt.

8. Verfahren nach einem der Ansprüche 6 oder 7, **dadurch gekennzeichnet, dass** die mindestens eine Kommunikationsvorrichtung nach einem der Ansprüche 1 bis 4 zum Erfassen von Daten ausgelegt ist, wobei die von der mindestens einen Kommunikationsvorrichtung erfassten Daten mindestens einen Parameter des fluidischen Gesamtsystems und / oder einen Parameter einer in dem fluidischen Gesamtsystem, insbesondere in der die Vorrichtungen miteinander fluidisch verbindenden Leitung, geführten Flüssigkeit widerspiegeln.

9. Verfahren nach einem der Ansprüche 6 bis 8, **dadurch gekennzeichnet, dass** das Verfahren auf ein fluidisches Gesamtsystem angewandt wird, welches mindestens eine Wasseraufbereitungsanlage und / oder mindestens eine Behandlungsmaschine, insbesondere eine Blutbehandlungsmaschine, und / oder mindestens eine Leitung, insbesondere eine Zufuhrleitung, eine Ringleitung, eine Wasserleitung, eine Dialysatleitung oder eine Konzentratleitung, aufweist.

10. Verfahren nach einem der Ansprüche 6 bis 9, **dadurch gekennzeichnet, dass** eine Mehrzahl von Kommunikationsvorrichtungen nach einem der Ansprüche 1 bis 4 an verschiedenen Positionen des fluidischen Gesamtsystems angeordnet werden.

11. System zur nicht-elektrischen Kommunikation zwischen miteinander fluidisch verbundenen Vorrichtungen, **gekennzeichnet durch** mindestens eine Kommunikationsvorrichtung nach einem der Ansprüche 1 bis 4, welche an mindestens einer Position des fluidischen Gesamtsystems angeordnet ist und dazu ausgelegt ist, über mindestens eine die Vorrichtungen fluidisch miteinander verbindende Leitung, vorzugsweise einer in dieser Leitung geführten Flüssigkeit, nicht-elektrisch, mittels Drucksignalen zu kommunizieren .

12. System nach Anspruch 11, **dadurch gekennzeichnet, dass** das System mindestens eine Wasseraufbereitungsanlage und mindestens eine Behandlungsmaschine, insbesondere eine Blutbehandlungsmaschine, und mindestens eine Leitung, insbesondere eine Zufuhrleitung, eine Ringleitung, eine Wasserleitung, eine Dialysatleitung oder eine Konzentratleitung, aufweist, welche miteinander fluidisch verbunden sind und so vorzugsweise ein fluidisches Gesamtsystem bilden.

## Claims

1. A communication device (5) for non-electric communication between devices fluidically connected to one another, **characterized in that** the communication device is adapted to be arranged in a position in a total fluidic system formed by the devices fluidically connected to one another and to receive and/or transmit non-electric signals, in the form of pressure signals, with the non-electric signals being transmitted over a line fluidically connecting the devices to one another and with the communication device being portable and adapted for online operation.

2. A communication device in accordance with claim 1, **characterized in that** the communication device has a pressure pulse generator for transmitting non-electric signals.

3. A communication device in accordance with one of the preceding claims, **characterized in that** the communication device is furthermore configured for detecting data with respect to at least one parameter of the total fluidic system and/or with respect to a parameter of a fluid conducted in the total fluidic system, in particular in the line fluidically connecting the devices to one another.

4. A communication device in accordance with claim 3, **characterized in that** the communication device has a sensor system for detecting data with respect to the at least one parameter, with the parameter preferably reflecting the conductivity, temperature, the flow volume, turbidity, or the transparency of the liquid, and the parameter of the total fluidic system, for example, reflecting an operating state or a maintenance state.

5. A kit for diagnosing a fluidic system comprising at least one communication device, preferably a plurality of communication devices, in accordance with one of the preceding claims.

6. A method of non-electric communication between devices that are fluidically connected to one another, **characterized in that** the non-electric communication takes place by means of pressure signals over at least one line fluidically connecting the devices, with at least one communication device in accordance with one of the claims 1 to 4 being arranged at at least one position in a total fluidic system formed by the devices that are fluidically connected to one another, said communication device preferably being configured for detecting data and/or for receiving non-electric signals and/or for transmitting non-electric signals.

7. A method in accordance with claim 6, **characterized in that** the non-electric communication takes place via a liquid conducted in the line fluidically connecting the devices.

8. A method in accordance with one of the claims 6 or 7, **characterized in that** the at least one communication device in accordance with one of the claims 1 to 4 is configured for detecting data, with the data detected by the at least one communication device reflecting at least one parameter of the total fluidic system and/or a parameter of a fluid conducted in the total fluidic system, in particular in the line fluidically connecting the devices to one another.

9. A method in accordance with one of the claims 6 to 8, **characterized in that** the method is applied to a total fluidic system that has at least one water preparation system, and/or at least one treatment machine, in particular a blood treatment machine, and/or at least one line, in particular a supply line, a loop, a water line, a dialyzate line, or a concentrate line.

10. A method in accordance with one of the claims 6 to 9, **characterized in that** a plurality of communication devices in accordance with one of the claims 1 to 4 are arranged at different positions of the total fluidic system.

11. A system for non-electric communication between devices that are fluidically connected to one another, **characterized by** at least one communication device in accordance with one of the claims 1 to 4 that is arranged at at least one position of the total fluidic system and that is configured to communicate non-electrically by means of pressure signals over at least one line fluidically connecting the devices to one another, preferably by means of a liquid conducted in this line.

12. A system in accordance with claim 11, **characterized in that** the system has at least one water preparation system and at least one treatment machine, in particular a blood treatment machine, and at least one line, in particular a supply line, a loop, a water line, a dialyzate line, or a concentrate line that are fluidically connected to one another and thus preferably form a total fluidic system.

## Revendications

1. Dispositif de communication (5) pour la communication non électrique entre des dispositifs en liaison fluidique les uns avec les autres, **caractérisé en ce que** le dispositif de communication est conçu pour être disposé dans une position dans un système fluidique global formé par les dispositifs en liaison fluidiques les uns avec les autres et recevoir et/ou envoyer des signaux non électriques sous la forme de signaux de pression, les signaux non électriques étant transmis par le biais d'une conduite reliant les dispositifs les uns aux autres de manière fluidique, et le dispositif de communication étant conçu portable et pour un fonctionnement en ligne.

2. Dispositif de communication selon la revendication 1, **caractérisé en ce que** le dispositif de communication présente, pour envoyer des signaux non électriques, un transmetteur d'impulsions de pression.

3. Dispositif de communication selon l'une des revendications précédentes, **caractérisé en ce que** le dispositif de communication est en outre conçu pour détecter des données concernant au moins un paramètre du système fluidique global et/ou un paramètre d'un liquide guidé dans le système fluidique global, en particulier dans la conduite reliant les dispositifs les uns aux autres de manière fluidique.

4. Dispositif de communication selon la revendication 3, **caractérisé en ce que** le dispositif de communication présente un système de capteurs pour détecter des données concernant l'au moins un paramètre, le paramètre reflétant de préférence la conductivité, la température, le débit volumique, la turbidité ou la transparence du liquide et le paramètre du système fluidique global reflétant par exemple un état de fonctionnement ou un état de maintenance.

5. Kit de diagnostic d'un système fluidique comprenant au moins un dispositif de communication, de préférence plusieurs dispositifs de communication, selon l'une des revendications précédentes.

6. Procédé de communication non électrique entre des dispositifs en liaison fluidique les uns avec les autres, **caractérisé en ce que** la communication non électrique s'effectue au moyen de signaux de pression par le biais d'au moins une conduite reliant les dispositifs de manière fluidique, au moins un dispositif de communication selon l'une des revendications 1 à 4 étant disposé en au moins une position dans un système fluidique global formé par les dispositifs en liaison fluidique les uns avec les autres, ledit dispositif de communication étant de préférence conçu pour détecter des données et/ou recevoir des signaux non électriques et/ou envoyer des signaux non électriques.

7. Procédé selon la revendication 6, **caractérisé en ce que** la communication non électrique s'effectue par le biais d'un liquide guidé dans la conduite reliant les dispositifs de manière fluidique.

8. Procédé selon l'une des revendications 6 ou 7, **caractérisé en ce que** l'au moins un dispositif de communication selon l'une des revendications 1 à 4 est conçu pour détecter des données, les données détectées par l'au moins un dispositif de communication reflétant au moins un paramètre du système fluidique global et/ou un paramètre d'un liquide guidé dans le système fluidique global, en particulier dans la conduite reliant les dispositifs les uns aux autres de manière fluidique.

9. Procédé selon l'une des revendications 6 à 8, **caractérisé en ce que** le procédé est appliqué à un système fluidique global qui présente au moins une installation de traitement de l'eau et/ou au moins une machine de traitement, en particulier une machine de traitement du sang, et/ou au moins une conduite, en particulier une conduite d'alimentation, une conduite en boucle, une conduite d'eau, une conduite de dialysat ou une conduite de concentré.

10. Procédé selon l'une des revendications 6 à 9, **caractérisé en ce qu'**une pluralité de dispositifs de communication selon l'une des revendications 1 à 4 sont disposés en différentes positions du système fluidique global.

11. Système de communication non électrique entre des dispositifs en liaison fluidique les uns avec les autres, **caractérisé par** au moins un dispositif de communication selon l'une des revendications 1 à 4, qui est disposé en au moins une position du système fluidique global et conçu pour communiquer de manière non électrique, au moyen de signaux de pression, par le biais d'au moins une conduite reliant les dispositifs les uns aux autres de manière fluidique, de préférence un liquide guidé dans cette conduite.

12. Système selon la revendication 11, **caractérisé en ce que** le système présente au moins une installation de traitement de l'eau et au moins une machine de traitement, en particulier une machine de traitement du sang, et au moins une conduite, en particulier une conduite d'alimentation, une conduite en boucle, une conduite d'eau, une conduite de dialysat ou une conduite de concentré, qui sont en liaison fluidique les unes avec les autres et forment ainsi de préférence un système fluidique global.
